# EUROPÄISCHE PATENTANMELDUNG

(11) **EP 2 362 207 A1**
(43) Veröffentlichungstag der Anmeldung: **31.08.2011**
(21) Anmeldenummer: 10152018.7
(22) Anmeldetag: 28.01.2010
(51) Int. Cl.: G01N 21/31, G01N 21/86, G01N 33/487

(54) **Messsystem und Messverfahren insbesondere zur Blutzuckerbestimmung**

(71) Anmelder: F. Hoffmann-La Roche AG, 4070 Basel (CH); Roche Diagnostics GmbH, 68305 Mannheim (DE)
(72) Erfinder: Hoenes, Joachim, 64673 Zwingenberg (DE); Wehowski, Frederic, 68766 Hockenheim (DE); Mischler, Reinhold, 67063 Ludwigshafen (DE); Harttig, Herbert, 67434 Neustadt (DE)
(74) Vertreter: Pfiz, Thomas

(57) **Zusammenfassung**

Die Erfindung betrifft ein Messsystem insbesondere zur Blutzuckerbestimmung mit einer photometrischen Messeinheit (16), die eine Lichtquelle (22) und einen Detektor (24) umfasst, und einem mit einer Probe, insbesondere einer Körperflüssigkeit beaufschlagbaren und für einen optischen Nachweis eines Analyten in einen Strahlengang (18) zwischen der Lichtquelle (22) und dem Detektor (24) bringbaren analytischen Testelement (14). Für eine verbesserte Mehrwellenlängenmessung wird vorgeschlagen, dass die Lichtquelle (22) einen in einem ersten Wellenlängenbereich zur Aussendung von pulsierendem Wechsellicht (28) ansteuerbaren ersten Strahler (26) und einen in einem zweiten Wellenlängenbereich zur Emission von Fluoreszenzlicht (32) anregbaren zweiten Strahler (30) aufweist.

## Beschreibung

Die Erfindung betrifft ein Messsystem insbesondere zur Blutzuckerbestimmung mit einer photometrischen Messeinheit, die eine Lichtquelle und einen Detektor umfasst, und einem mit einer Probe, insbesondere einer Körperflüssigkeit beaufschlagbaren und für einen optischen Nachweis eines Analyten in einen Strahlengang zwischen der Lichtquelle und dem Detektor bringbaren analytischen Testelement. Die Erfindung betrifft weiter ein entsprechendes Messverfahren.

In der Praxis für Blutzuckerbestimmungen bekannte Systeme dieser Art basieren auf irreversibel reagierenden trägergebundenen Testelementen in Form von Teststreifen oder Testbändern. Diese sollen auch dem Laien durch Verarbeitung in automatischen Testgeräten die Messung von Blutglucose außerhalb einer Laborumgebung mit hinreichender Genauigkeit ermöglichen, wie sie die medizinische Behandlung von Diabetes erfordert. Der Messablauf sieht vor, dass nach Aufbringen einer Blutprobe die Analytkonzentration durch zweckmäßig wiederholte photometrische Messungen ermittelt wird. Dabei ist es wichtig, dass Veränderungen in den Randbedingungen der Messung unabhängig von der eigentlichen Analyterfassung erkannt werden.

Ausgehend hiervon liegt der Erfindung die Aufgabe zugrunde, die im Stand der Technik bekannten Vorrichtungen und Verfahren und insbesondere die Qualität und Genauigkeit des Messprozesses weiter zu verbessern, wobei mit begrenztem Aufwand ein effizientes System geschaffen werden soll.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Dementsprechend wird im Hinblick auf ein Messsystem vorgeschlagen, dass die Lichtquelle einen in einem ersten Wellenlängenbereich zur Aussendung von pulsierendem Wechsellicht ansteuerbaren ersten Strahler und einen in einem zweiten Wellenlängenbereich zur Emission von Fluoreszenzlicht anregbaren zweiten Strahler aufweist. Damit lässt sich eine kompakte und effiziente Mehrwellenlängenlichtquelle realisieren, die regelbar Strahlung in verschiedenen Wellenlängenbereichen abgibt und eine selektive Signalauswertung gestattet. Somit ist es auch möglich, zeitgleich eine Analyt- und Kontrollmessung durchzuführen, um so die erforderliche Messqualität sicherstellen zu können.

Für eine selektive Signalerfassung ist es vorteilhaft wenn das Wechsellicht eine Impulsdauer aufweist und das Fluoreszenzlicht mit einer Fluoreszenzlebensdauer abklingt, und wenn die Fluoreszenzlebensdauer um ein Vielfaches größer als die Impulsdauer ist. Damit können unterschiedliche Lebensdauern bzw. Abklingzeiten in den verschiedenen Wellenlängenbereichen zur elektronischen Selektion anstelle einer aufwändigen Wellenlängenselektion mit Filtern oder ähnlichem genutzt werden.

Eine baulich vorteilhafte Ausführung sieht vor, dass der erste Strahler durch eine insbesondere im UV-Bereich emittierende Leuchtdiode gebildet ist, und dass der zweite Strahler durch einen Leuchtstoff gebildet ist, welcher durch den gepulsten ersten Strahler zur Abgabe von insbesondere sichtbarem Fluoreszenzlicht optisch anregbar ist. Leuchtdioden weisen hohe Leuchtdichten auf und können daher gut für intensive gebündelte Lichtstrahlen eingesetzt werden. Durch die Kombination mit einem Leuchtstoff kann auf mehrere Einzel-LEDs, die an verschiedenen Stellen sitzen und nur aufwändig zu einem homogenen Lichtstrahl gebündelt werden können, verzichtet werden.

Eine weitere messtechnische Verbesserung ergibt sich dadurch, dass der zweite Strahler als fluoreszierende Leuchtstoffschicht auf eine Abstrahlfläche des ersten Strahlers aufgebracht ist, so dass beide Strahler gemeinsam auf eine Messfläche des Testelements ausgerichtet sind.

Vorteilhafterweise weist der Detektor einen Photoempfänger zur gemeinsamen Erfassung des Wechsel- und Fluoreszenzlichts und zwei Verstärkerkanäle zur wellenlängenselektiven Messwertbestimmung auf.

Zur zeitaufgelösten separaten Erfassung eines durch das Wechsellicht erzeugten Wechsellicht-Signalanteils ist es vorteilhaft, wenn der Detektor einen mit der Impulsfrequenz des Wechsellichts modulierbaren Lock-in-Verstärker aufweist.

Für die Signalverarbeitung ist es von Vorteil, wenn der Detektor einen insbesondere als Integrator arbeitenden Verstärker zur Erfassung eines durch das Wechsellicht und Fluoreszenzlicht erzeugten Summensignals und einen Signalprozessor zur Bestimmung von Signalanteilen des Wechsellichts und/oder Fluoreszenzlichts, insbesondere zur Subtraktion des Wechsellicht-Signalanteils von dem Summensignal umfasst.

Für patientennahe Messungen vor Ort ist es günstig, wenn die Messeinheit in einem Handgerät integriert ist und das Testelement als Verbrauchsmittel für einen Einmaleinsatz in dem Handgerät ausgelegt ist.

Eine besonders vorteilhafte Ausführung sieht vor, dass das Testelement über eine Optikeinheit, insbesondere einen Lichtleiter an die Lichtquelle ankoppelbar ist, und dass die optische Ankopplung durch eine gesonderte Signalauswertung in einem der Wellenlängenbereiche kontrollierbar ist. Für die Einkoppelung in dünne Lichtleiter ist eine möglichst kleine Lichtquelle nötig, um hohe Einkoppeleffizienz zu erreichen. Hierbei ist anzumerken, dass sich mehrere gesonderte LEDs nur unter hohen Verlusten einkoppeln lassen.

In verfahrensmäßiger Hinsicht wird die eingangs genannte Aufgabe dadurch gelöst, dass ein erster Strahler der Lichtquelle in einem ersten Wellenlängenbereich zur Aussendung von pulsierendem Wechsellicht angesteuert wird, und dass ein zweiter Strahler in einem zweiten Wellenlängenbereich zur Emission von dem Wechsellicht überlagertem Fluoreszenzlicht angeregt wird.

Dabei kann durch eine zeitaufgelöste Signalerfassung vorzugsweise mittels eines Lock-in-Verstärkers ein dem Wechsellicht zugeordneter Signalanteil erfasst werden. Ein weiterer Vorteil ergibt sich dadurch, dass in einem Wellenlängenbereich ein Messwert für einen Analyten in der Probe und in dem anderen Wellenlängenbereich ein Kontrollwert für die optische Ankopplung des Testelements an die Messeinheit erfasst wird.

Im Folgenden wird die Erfindung anhand der in der Zeichnung schematisch dargestellten Ausführungsbeispiele näher erläutert. Es zeigen
- Fig. 1: ein Blockschaltbild eines Messsystems zur Blutzuckermessung mit einem Mehrwellenlängen-Photometer;
- Fig. 2: einen Zeitverlauf der Strahlungsintensität eines Impulsstrahlers und eines Fluoreszenzstrahlers des Photometers nach Fig. 1;
- Fig. 3: die Strahlungsintensität auf einer logarithmischen Zeitskala für ein weiteres Ausführungsbeispiel einer Mehrwellenlängenmessung.

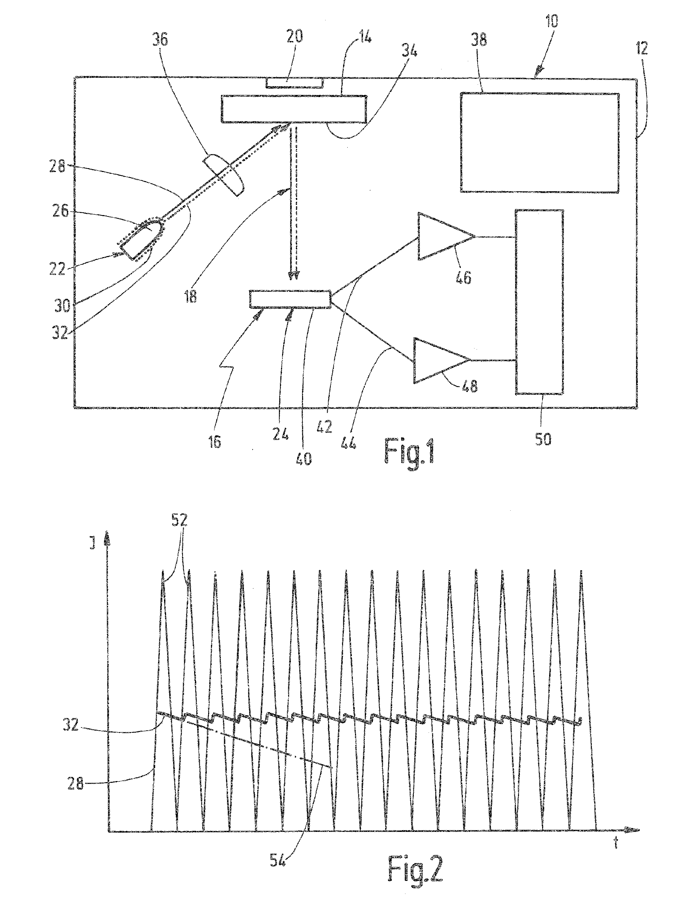

Das in Fig. 1 dargestellte Messsystem 10 umfasst ein tragbares Testgerät 12 und darin einsetzbare Testelemente 14 für jeweils einen Einmaltest an einer Probe, speziell zur Glukosebestimmung in einer Blutprobe. Zu diesem Zweck weist das Testgerät 12 eine photometrische Messeinheit 16 auf, in deren Strahlengang 18 ein disposibles Testelement 14 beispielsweise in Form eines Teststreifens oder Testbands einbringbar ist, wobei über eine Aufnahme 20 für ein Körperteil ein direkter Probenauftrag auf das Testelement 14 möglich ist. Das Testelement 14 ist mit einer trockenchemischen Reagenzschicht versehen, die auf einen Analyten (z.B. Glucose) durch einen photometrisch messbaren Farbumschlag reagiert.

Die photometrische Messeinheit 16 umfasst eine Mehrwellenlängenlichtquelle 22 und einen zweikanaligen Detektor 24. Ein erster Strahler 26 der Lichtquelle 22 ist zur Aussendung von pulsierendem Wechsellicht 28 ansteuerbar, während ein zweiter Strahler 30 durch den ersten Strahler 26 zur Emission von längerwelligem Fluoreszenzlicht 32 anregbar ist. Der erste Strahler 26 ist zweckmäßig durch eine im UV-Bereich emittierende Leuchtdiode und der zweite Strahler durch eine im sichtbaren Wellenlängenbereich fluoreszierende Leuchtstoffschicht auf der Leuchtdiode gebildet. Auf diese Weise sind beide Strahler 26, 28 als einheitliches Bauteil bestrombar und gemeinsam auf eine Messfläche 34 des Testelements 14 ausrichtbar. Zur Lichtbündelung auf einen möglichst kleinen Messfleck kann eine Sammellinse 36 und/oder ein Lichtleiter im optischen Übertragungsweg vorgesehen sein.

Um Zustandsänderungen der optischen Ankopplung des Testelements 14 zu erkennen und das optische Übertragungsverhalten zu überprüfen, kann mittels des Fluoreszenzlichts ein Kontrollwert bestimmt werden, während das auf den Analyten ansprechende UV-Licht die Bestimmung eines Messwerts ermöglicht, der auf dem Display 38 in Form einer digitalen Konzentrationsangabe für einen Benutzer anzeigbar ist.

Die Kontrollwertbestimmung ermöglicht es, eine unbeabsichtigte Beeinflussung der Messbedingungen durch eine geräteseitige Aktuation oder benutzerseitige Eingriffe im Zuge der Bereitstellung des Testelements zu erkennen. Beispielsweise kann der Probenauftrag durch Andrücken eines Körperteils auf das Testelement 14 zu einer ungewollten Verformung oder Verschiebung führen. Hinzu kommt, dass die Erfassung der Messwerte zum Startzeitpunkt der Probenapplikation unsicher bzw. ungenau ist, weil die Testfeldbenetzung nicht schlagartig homogen erfolgt und die optischen Eigenschaften sich ändern. Um hier Abhilfe zu schaffen, kann durch eine Zweiwellenlängenmessung die erforderliche Messgenauigkeit und Robustheit gegen Störungen mit geringem apparativem Aufwand sichergestellt werden.

Zu diesem Zweck weist der Detektor 24 einen in beiden Messwellenlängenbereichen empfindlichen Photoempfänger 40 und zwei daran angeschlossene Verstärkerstufen bzw. -kanäle 42, 44 zur wellenlängenselektiven Messwertbestimmung auf. In der ersten Verstärkerstufe 42 ist ein Lock-in-Verstärker 46 zur zeitaufgelösten Erfassung eines Wechsellicht-Signalanteils angeordnet. In der zweiten Verstärkerstufe befindet sich ein integrierender Verstärker 48 zur integralen Erfassung eines durch das Wechsellicht und das Fluoreszenzlicht erzeugten Summensignal. Ein nachgeordneter Signalprozessor 50 ermöglicht die Subtraktion des Wechsellicht-Signalanteils von dem Summensignal und somit die separate Bestimmung des Fluoreszenzlicht- bzw. Gleichlicht-Signalanteils.

Wie aus Fig. 2 ersichtlich, kann der erste Strahler 26 durch schnelle Strompulse beispielsweise im Mikrosekundentakt zur Aussendung von pulsierendem Wechsellicht angesteuert werden. Das UV-Licht 28 wird durch die Stromimpulse nahezu verzögerungsfrei erzeugt, so dass sich einzeln auflösbare Lichtimpulse 52 mit einer Impulsdauer von 1 µs ergeben. Das dadurch angeregte Fluoreszenzlicht des zweiten Strahlers kann diesem Rhythmus jedoch nicht folgen, da es mit einer wesentlich längeren Zeitkonstante abklingt. In dem gezeigten Beispiel ist durch die strichpunktierte Kurve 54 der Signalabfall für einen Fluorophor als Leuchtschicht 30 mit einer Fluoreszenzlebensdauer von z.B. 20 µs verdeutlicht. Beispielhaft zu nennen ist hier der im Handel unter der Markenbezeichnung Lumilux CD163 von der Firma Honeywell erhältliche Fluorophor, der im grünen Wellenlängenbereich bei max. 517 nm fluoresziert. Aufgrund der schnellen Impulsfolge wird der Signalabfall entsprechend verkürzt, so dass ein Gleichlichtanteil mit nur geringer Restwelligkeit beobachtbar ist. Somit ergibt sich also ein schneller Wechsel des kurzwelligen Lichts 28 überlagert durch fast konstantes Gleichlicht des längerwelligen Fluoreszenzlichts 32.

Die Messung des Wechsellichtanteils ist mit dem Lock-in-Verstärker 46 auf einfache Weise möglich. Hierbei wird ein Referenzsignal mit der Frequenz des modulierten UV-Lichts erzeugt und durch einen Phasenschieber in seiner Phasenlage angepasst. Alternativ zu einem Phasenschieber kann auch ein so genannter Double-lock-Verstärker eingesetzt werden, der durch eine Doppelmessung bei den Phasenlagen 0 und 90 Grad das Wechsellicht phasenunabhängig erfassen kann. Durch einen Multiplizierer wird dann das eigentliche Messsignal mit dem Referenzsignal multipliziert, so dass nur der Wechsellichtanteil ein endliches Ausgangssignal liefert und der Hauptteil des grünen Gleichlichts nicht miterfasst wird.

Eine Signalintegration mittels des Verstärkers 48 über längere Zeit ergibt die Summe von UV-Licht 28 und grünem Licht 32. Sodann kann ein einfacher Algorithmus in dem Signalprozessor 50 den Wechsellichtanteil aus dem Summensignal subtrahieren, um so einen separaten Messwert für den Gleichlichtanteil zu erhalten.

Grundsätzlich ist es nicht erforderlich, Wechsellicht und Gleichlicht mit sehr stark unterschiedlichen Frequenzen völlig getrennt zu erfassen. Auch bei näher zusammen liegenden Frequenzen ergeben sich zwei unterschiedliche Signale mit unterschiedlichen Anteilen der beiden Wellenlängen, die sich durch Lösen eines Gleichungssystems (zwei Gleichungen mit zwei Unbekannten) ermitteln lassen. Der Integrator kann durch einen Verstärker mit geeigneter anderer Frequenz ersetzt werden, und der Prozessor muss nicht auf eine einfache Subtraktion beschränkt sein.

Wie aus der folgenden Tabelle schematisch hervorgeht, lässt sich das erläuterte Mehrwellenlängen-Messprinzip mit geeigneten Fluorophoren abgestufter Abklingzeiten auch auf mehr als zwei Wellenlängen bzw. Lichtfarben ausweiten. Als Grenzfrequenz für das Erreichen einer hohen Pulsamplitude ist hierbei die Frequenz angegeben, die der zehnfachen Abklingzeit entspricht.

| Wellenlänge | Abklingzeit | Grenzfrequenz |
|---|---|---|
| Ultraviolett | 10ns (LED) | 10 MHz |
| Blau | 1µs (LUMILUX Blau) | 100 kHz |
| Grün | 100µs (LUMILUX Grün) | 1 kHz |

Das Wechsellicht von 10 MHz enthält somit nur die UV-Intensität, das Wechsellicht von 100 kHz die Summe von UV und Blau, während bei Frequenzen von 1 kHz und kleiner die gesamte Lichtintensität erfasst wird.

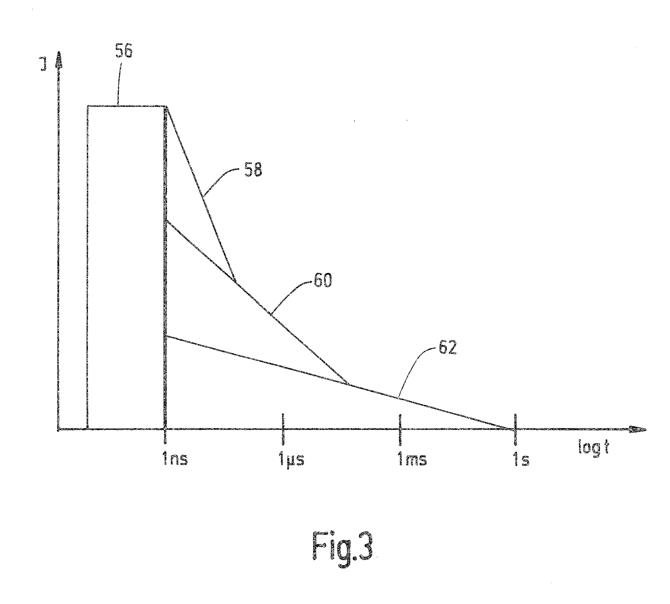

Die Messung lässt sich auch gemäß Fig. 3 durchführen, indem mittels eines Stromimpulses ein rechteckförmiger Lichtimpuls 56 erzeugt und dessen Abklingen analysiert wird. In den einzelnen Zeitbereichen der logarithmischen Zeitskala lässt sich jeweils das Abklingen eines Wellenlängenbereiches 58, 60, 62 separat erfassen. Dieses Verfahren entspricht dem Messverfahren, das bei der Bestimmung von Fluoreszenzlebensdauern an sich bekannt ist. Die exponentielle Abklingkurve wird dabei mit einer, zwei oder drei Fluoreszenzlebensdauern und dazugehörigen Intensitätsanteilen als Fitparameter angepasst. In der vorliegenden Anwendung sind die charakteristischen Lebensdauern in den drei Wellenlängenbereichen 58, 60, 62 von vornherein bekannt. Daher kann ein Fit mit nur drei Parametern, nämlich den drei Intensitätsanteilen in den jeweiligen Wellenlängenbereichen, durchgeführt werden. Die UV-Strahlung folgt dem Abklingen der LED, die Abklingkurven der Fluorophore folgen den Fluoreszenzlebensdauern, die zweckmäßigerweise hinreichend unterschiedlich gewählt sind, beispielsweise um einen Faktor von mindestens 3. Hierbei ist abzuwägen zwischen der Möglichkeit zur schärferen Wellenlängendifferenzierung durch größere Lebensdauerunterschiede und einer verlängerten Messzeit bei verlängerter Abklingdauer.

## Patentansprüche

1. Messsystem insbesondere zur Blutzuckerbestimmung mit einer photometrischen Messeinheit (16), die eine Lichtquelle (22) und einen Detektor (24) umfasst, und einem mit einer Probe, insbesondere einer Körperflüssigkeit beaufschlagbaren und für einen optischen Nachweis eines Analyten in einen Strahlengang (18) zwischen der Lichtquelle (22) und dem Detektor (24) bringbaren analytischen Testelement (14), gekennzeichnet, dass die Lichtquelle (22) einen in einem ersten Wellenlängenbereich zur Aussendung von pulsierendem Wechsellicht (28) ansteuerbaren ersten Strahler (26) und einen in einem zweiten Wellenlängenbereich zur Emission von Fluoreszenzlicht (32) anregbaren zweiten Strahler (30) aufweist.

2. Messsystem nach Anspruch 1, **dadurch gekennzeichnet, dass** das Wechsellicht (28) eine Impulsdauer aufweist und das Fluoreszenzlicht (32) mit einer Fluoreszenzlebensdauer abklingt, und dass die Fluoreszenzlebensdauer um ein Vielfaches größer als die Impulsdauer ist.

3. Messsystem nach Anspruch 1 oder 2, **dadurch gekennzeichnet, dass** der erste Strahler (26) durch eine insbesondere im UV-Bereich emittierende Leuchtdiode gebildet ist.

4. Messsystem nach einem der Ansprüche 1 bis 3, dagekennzeichnet, dass der zweite Strahler (30) durch einen Leuchtstoff gebildet ist, welcher durch den gepulsten ersten Strahler (26) zur Abgabe von insbesondere sichtbarem Fluoreszenzlicht (32) optisch anregbar ist.

5. Messsystem nach einem der Ansprüche 1 bis 4, **dadurch gekennzeichnet, dass** der zweite Strahler (30) als fluoreszierende Leuchtstoffschicht auf eine Abstrahlfläche des ersten Strahlers (26) aufgebracht ist, so dass beide Strahler gemeinsam auf eine Messfläche (34) des Testelements (14) ausgerichtet sind.

6. Messsystem nach einem der Ansprüche 1 bis 5, **dadurch gekennzeichnet, dass** der Detektor (24) einen Photoempfänger (40) zur gemeinsamen Erfassung des Wechsel- und Fluoreszenzlichts (28,32) aufweist.

7. Messsystem nach einem der Ansprüche 1 bis 6, dagekennzeichnet, dass der Detektor (24) zwei Verstärkerkanäle (42,44) zur wellenlängenselektiven Messwertbestimmung aufweist.

8. Messsystem nach einem der Ansprüche 1 bis 7, **dadurch gekennzeichnet, dass** der Detektor (24) einen mit der Impulsfrequenz des Wechsellichts (28) modulierbaren Lock-in-Verstärker (46) zur Erfassung eines durch das Wechsellicht (28) erzeugten Wechsellicht-Signalanteils aufweist.

9. Messsystem nach einem der Ansprüche 1 bis 8, dagekennzeichnet, dass der Detektor (24) einen insbesondere als Integrator (48) arbeitenden Verstärker zur Erfassung eines durch das Wechsellicht (28) und Fluoreszenzlicht (32) erzeugten Summensignals umfasst.

10. Messsystem nach einem der Ansprüche 1 bis 9, **dadurch gekennzeichnet, dass** der Detektor (24) einen Signalprozessor (50) zur Bestimmung von Signalanteilen des Wechsellichts und/oder Fluoreszenzlichts, insbesondere zur Subtraktion des Wechsellicht-Signalanteils von dem Summensignal aufweist.

11. Messsystem nach einem der Ansprüche 1 bis 10, **dadurch gekennzeichnet, dass** die Messeinheit (16) in einem Handgerät (12) integriert ist und das Testelement (14) als Verbrauchsmittel für einen Einmaleinsatz in dem Handgerät (12) ausgelegt ist.

12. Messsystem nach einem der Ansprüche 1 bis 11, **dadurch gekennzeichnet, dass** das Testelement (14) über eine Optikeinheit (36), insbesondere Linse oder einen Lichtleiter an die Lichtquelle (22) ankoppelbar ist, und dass die optische Ankopplung durch eine gesonderte Signalauswertung in einem der Wellenlängenbereiche kontrollierbar ist.

13. Messverfahren insbesondere zur Blutzuckerbestimmung bei welchem eine Probe, insbesondere eine Körperflüssigkeit auf ein als Verbrauchsmittel einsetzbares analytisches Testelement (14) aufgebracht wird und das Testelement (14) mittels einer eine Lichtquelle (22) und einen Detektor (24) umfassenden photometrischen Messeinheit (16) optisch abgetastet wird, **dadurch gekennzeichnet, dass** ein erster Strahler (26) der Lichtquelle (22) in einem ersten Wellenlängenbereich zur Aussendung von pulsierendem Wechsellicht (28) angesteuert wird, und dass ein zweiter Strahler (30) in einem zweiten Wellenlängenbereich zur Emission von dem Wechsellicht (28) überlagertem Fluoreszenzlicht (32) angeregt wird.

14. Messverfahren nach Anspruch 13, **dadurch gekennzeichnet, dass** durch eine zeitaufgelöste Signalerfassung vorzugsweise mittels eines Lock-in-Verstärkers (46) ein dem wechsellicht (28) zugeordneter Signalanteil ermittelt wird.

15. Messverfahren nach Anspruch 13 oder 14, **dadurch gekennzeichnet, dass** in einem Wellenlängenbereich ein Messwert für einen Analyten in der Probe und in dem anderen Wellenlängenbereich ein Kontrollwert für die optische Ankopplung des Testelements (14) an die Messeinheit (16) erfasst wird.
